(19) **Europäisches Patentamt / European Patent Office / Office européen des brevets**

(11) **EP 4 308 001 B1**

(12) **EUROPEAN PATENT SPECIFICATION**

(45) Date of publication and mention
of the grant of the patent:
**19.11.2025 Bulletin 2025/47**

(21) Application number: **22709333.3**

(22) Date of filing: **09.03.2022**

(51) International Patent Classification (IPC):
***A61B 5/305*** (2021.01)   ***A61B 5/318*** (2021.01)
***A61B 5/00*** (2006.01)

(52) Cooperative Patent Classification (CPC):
**A61B 5/305; A61B 5/318; A61B 5/725**

(86) International application number:
**PCT/EP2022/056001**

(87) International publication number:
**WO 2022/194632 (22.09.2022 Gazette 2022/38)**

(54) **PHYSIOLOGICAL MEASUREMENT DEVICE COMPRISING ADAPTIVE FILTERS AND METHOD**

PHYSIOLOGISCHE MESSVORRICHTUNG AUFWEISEND ADAPTIVE FILTER UND VERFAHREN

DISPOSITIF DE MESURE PHYSIOLOGIQUE COMPRENANT DES FILTRES ADAPTIFS ET PROCÉDÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.03.2021 EP 21162472**

(43) Date of publication of application:
**24.01.2024 Bulletin 2024/04**

(73) Proprietor: **Koninklijke Philips N.V.
5656 AG Eindhoven (NL)**

(72) Inventor: **FRANCK, Christoph Florian
5656 AG Eindhoven (NL)**

(74) Representative: **Philips Intellectual Property &
Standards
High Tech Campus 52
5656 AG Eindhoven (NL)**

(56) References cited:
**EP-A1- 2 394 571     EP-A1- 3 372 148
WO-A1-2014/021886     US-A- 4 793 361**

• **ADAM D ET AL: "COMPLETE FOETAL ECG
MORPHOLOGY RECORDING BY
SYNCHRONISED ADAPTIVE FILTRATION",
MEDICAL AND BIOLOGICAL ENGINEERING AND
COMPUTING, SPRINGER, HEILDELBERG, DE,
vol. 28, no. 4, 1 July 1990 (1990-07-01), pages 287
- 292, XP000136090, ISSN: 0140-0118, DOI:
10.1007/BF02446144**

**Description**

FIELD OF THE INVENTION

The present invention relates to a physiological measurement device and a respective method. Such device may comprise a plurality of input channels, at least one of the input channels including a digital section configured to process at least one digital signal representing an analog signal present at an electrode assigned to the respective channel and a signal combiner configured to calculate at least one vector signal from at least two processed digital signals.

BACKGROUND OF THE INVENTION

The combiner of the above-named device combines the at least processed digital signal to the vector signal so that common mode interferences present in the processed digital signals are mitigated.

A known issue when performing electrophysiological measurements such as electrocardiography (ECG), Electroencephalography (EEG) Electromyography (EMG) is that a signal path of each electrode has a slightly different transfer function due to component tolerances, different external cables, and the electrical properties of the skin-electrode interface, etc. As a consequence, the common-mode signal can be cancelled only partially.

The published patent application DE 10 2014 214 994 A1 describes a differential voltage measurement system that comprises an analog variable impedance element such as an RC-circuit that can be controlled for the purpose of impedance matching of two inputs of the measurement system. Moreover, combination of this impedance matching approach with a Right Leg Drive (RLD) technique is proposed to further reduce common mode interference.

EP 3372148 A1 discloses systems, methods, and apparatuses for reducing interference of signals transmitted by a physiological measurement device, such as an electrocardiogram device. The physiological measurement device can employ filters that use coefficients to reduce time-domain differences between response signals of the physiological measurement device. The coefficients can be derived during a calibration process where each channel of the physiological measurement device is supplied a test signal for identifying the channel with the slowest or most delayed response. Thereafter, when a monitor signal is compiled from response signals filtered using the coefficients, differences in timing between the response signals will not result in distortion of the monitor signal, thereby rendering the monitor signal more accurate for measurement purposes.

EP 2,394,571 A1 discloses an apparatus and method for measuring a biological signal.

Adam et al. 1990. "Complete foetal ECG morphology recording by synchronised adaptive filtration". Medical and Biological Engineering and Computing, 28(4):287-292 discloses a technique that uses time-sequence adaptive filters to reject maternal ECG and enhance fetal ECG.

US 4.793.361 A discloses a method and apparatus for detecting the P-wave component in a surface electrocardiograph .

WO 2014 021886 A1 discloses a system and method for determining at least one patient parameter using electrophysiological signals from the patient.

SUMMARY OF THE INVENTION

It is an object of the present invention to provide a physiological measurement device, a physiological measurement method and a respective computer program product that allow for improved common-mode interference mitigation. Moreover, a cost-efficient implementation of a signal acquisition front-end should be possible without compromising the effectiveness of common-mode interference mitigation.

In a first aspect of the present invention a physiological measurement device is presented as defined in claim 1.

In further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed device, method, computer program and medium have similar and/or identical preferred embodiments as the claimed system, in particular as defined in the dependent claims and as disclosed herein.

The present invention is based on the idea that characteristics of the vector signal can be considered for adapting the digital filter of a channel of the measurement device rather than focusing on channel-specific characteristics like impedance. By adapting the set of coefficients of at least one digital filter of one channel based on the characteristics of the vector signal, the common-mode interference can be particularly well mitigated. As a consequence, medical personnel operating the measurement device will see cleaner measured curves (e.g. EEG or ECG curves) having less disturbing noise.

The use of digital filtering allows for compensating time skew between digital signals of different channels. Such time skew may be introduced by cost-efficient Analog to Digital Converter (ADC) architectures that use a single ADC to subsequently digitize multiple analog signals related to different channels. **In** other words, the claimed invention allows to implement a cost-efficient signal acquisition frontend without compromising common-mode mitigation effectiveness. The claimed device, method and program operate so effectively that the RLD technique or similar methods are not needed in order to archive good measurement results.

In an exemplary implementation, calculating the set of filter coefficients may be based on the unfiltered digital signal or samples thereof.

Moreover, calculating the set of filter coefficients can be based on a current set of filter coefficients. When calculating one or more new sets of filter coefficients for one or more digital filters based on the unfiltered digital signal and the current set of filter coefficients. An improvement step can be performed to adjust the currently used filter coefficients. This improvement step may be carried out during normal operation of the measurement device, e.g. during acquisition of a patient's ECG, EEG or EMG curves. Separate calibration or usage of special measurement signal is not needed.

Preferably, calculating the set of filter coefficients is performed repeatedly for each newly available sample of the digital signal or for a block of multiple subsequent samples of the digital signal. During operation of the measurement device, the common-mode rejection behavior will thus improve gradually while the filter coefficients are successively adjusted.

The improvement step may, e.g., include an optimization run. In other words, calculating the set of filter coefficients may comprise performing an optimization step, the measure constituting a target function and the set of filter coefficients constituting input variables of the target function. Although the herein described examples mainly use a cost function to be minimized - the target function may thus be a cost function - other types of target function may be applied, e.g. a utility function that needs to be maximized.

The inventor has realized that measures, in particular statistical measures, can described features of the vector signal that allow for distinguishing between a desired physiological measurement signal and common-mode interference. To allow for improved distinguishing the desired signal and the common-mode interference from each other, it is possible that the target function comprises a weighted sum of multiple measures.

To exclude undesired digital filter configurations form the optimization solution space, in a certain implementation, performing the optimization step may be subject to at least one constraint related to a certain set of filter coefficients. Such constraint may includes at least one equality constraint and/or at least one inequality constraint.

To avoid solutions where, e.g., the common-mode inference is filtered by the digital filters rather than eliminated by the signal combiner, the optimization step may be subject to at least one constraint related to a similarity measure characterizing a similarity of two different sets of filter coefficients.

Alternatively or in addition to the constraint related to the similarity measure, the target function may comprise the similarity measure characterizing a similarity of two different sets of filter coefficients.

Preferably, the processor may be configured to calculate multiple vector signals from the filtered digital signals, wherein the digital section of one channel includes at most one digital filter. The individual digital filters are thus specific to one channel but not necessarily specific to a certain vector signal.

In an exemplary implementation, the processor is configured to receive an indication from a back end and to determine the at least one measure and/or the target function based on the received indication. The back end may be part of the measurement device or included into a separate device. The indication allows to adapt the measurement device to special measurement situation that may be automatically detected by the back end and/or determined by the back end based on human user input. This adaptation may include selecting a target function and/or or composing a target function based on a set multiple measure. In particular, the weights of the weights sum of the multiple measures may be determined based on the indication.

For example, the signal combiner may be configured to calculate the vector signal from a further vector signal ($x_3$). In an ECG application, the further vector signal may correspond to a Wilson Central Terminal (WCT). The at least one vector signal calculated from the further vector signal may correspond to a lead related to a chest electrode.

The measurement device may include a signal acquisition frontend that includes the individual input channel comprising analog input circuitry including analog filters, ADC, the digital filters, the signal combiner and the processor. Optionally, the back end may also be included into the measurement device. The measurement device may be an ECG, an Electroencephalography (EEG), or an Electromyography (EMG) device for medical (diagnosis, patient monitoring, etc.) or non-medical (fitness trackers, smart watches, etc.) applications.

The input signals present at the electrodes of the different channels are generally sensed (acquired or measured) from a patient during use of the device, i.e., the digital section is configured to process a digital signal representing an analog signal present at an electrode assigned to the respective channel and sensed from a patient during use of the device. The measurement device may hence be configured to obtain (receive or retrieve) the input signals from the electrodes or sense the input signal via the electrodes at the patient.

BRIEF DESCRIPTION OF THE DRAWINGS

These and other aspects of the invention will be apparent from and elucidated with reference to the embodiment(s) described hereinafter. In the following drawings

Fig. 1 shows a block diagram of a physiological measurement device according to a first example;
Fig. 2 shows a flowchart of a method to operate the device of Fig. 1;
Fig. 3 shows an ECG measurement device according to a second example and electrodes connected to this device;
Fig. 4 shows a block diagram of an ECG signal acquisition content of the measurement device shown in Fig. 3;
Fig. 5 shows a block diagram of a ECG signal acquisition signal front end according to a third example; and
Fig. 6 shows a diagram of signals within a measurement device over time.

DETAILED DESCRIPTION OF EMBODIMENTS

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

Electrophysiological measurements like electrocardiography, electroencephalography and electromyography work by measuring voltages that appear between certain points on a patient due to physiological cellular activity. The signals of interest (wanted signals or desired signals) are usually small - in the millivolt to microvolt range - and typically only the difference in electrical potential between the electrode positions is relevant. However, due to factors like electromagnetic interference (e.g. from power lines or electrical devices in close proximity of or in contact with the patient), the electrical potential of the patient changes over time, which leads each electrode picking up a mixture of the differential-mode physiological signal of interest and a non-physiological, undesired common-mode signal.

The difference between two electrode locations, or more general, any linear combination of electrode potentials where the sum of weighting coefficients is zero, is usually referred to as a vector signal or just a "vector". In the ideal case, the common-mode signal disappears completely when the potential difference between two electrodes is formed by calculating the vector signal.

However, in a real implementations, each electrode has a slightly different transfer function, which is defined by the quality of the patient/electrode interface, the properties of the cable between the electrode and the measurement device, component tolerances in the input circuitry of the measurement device, and the properties of the analog-to-digital conversion like time skew (in case of sequential sampling) and slightly different transfer functions if multiple analog-to-digital converters (ADCs) are used. The differences in the transfer functions can lead to a significant part of the undesired common-mode signal appearing in the vector as what is called common-mode interference.

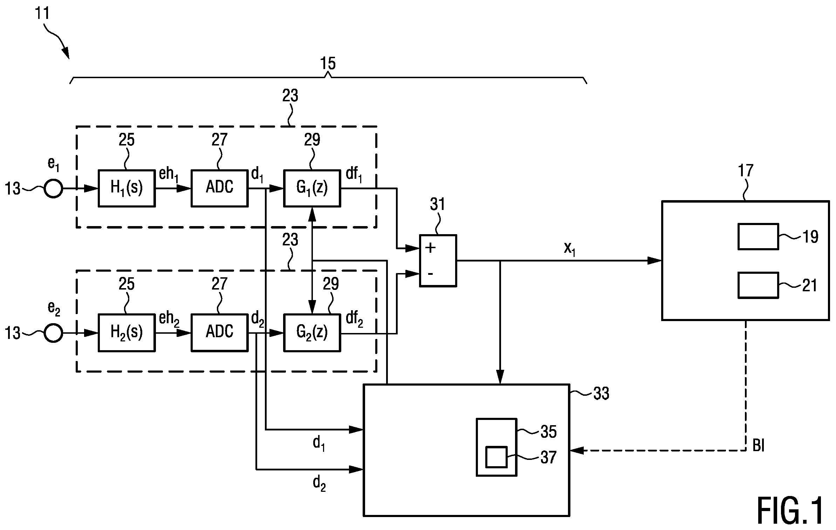

Fig. 1 shows a block diagram of a simple electrophysiological measurement device 11 having two electrodes 13. The measurement device 11 may comprise a signal acquisition front end 15 and an analysis back end 17. The front end 15 is configured to process analog signals $E_1$, $E_2$ inputted by the front end 15 via the electrodes 13 and to generate a vector signal x therefrom.

The back end 17 is operable for receiving the vector signal x from the front end 15 and therefrom further operations such as further signal analyses and/or signal classification, in particular rhythm classification in case of a ECG device. Moreover, the back end 17 may include user interface elements such as a screen and/or a keyboard, or the like to display the vector signal x in original or post process form or to display signal analysis or classification results to a user of the device 11. The analysis back end 17 may comprise a back end processor 21, which may include a computer program to perform the above-mentioned operations of the back end 17. Other than the shown example, a measurement device may be provided that does not include the back end 17. For example, the back end may be remote with respect to the measurement device.

The front end 15 comprises input channels 23 dedicated to the individual electrodes 13. The exemplary device shown in Fig. 1 has two input channels 23 that can be connected to two electrodes 13. The disclosure of the present patent application is not limited to this comparatively simple example. It can be used in connection with measurement devices having a larger number of input channels 23 and thus supporting a higher number of connected electrodes 13.

Each input channel 23, includes an analog section comprising circuitry for inputting the analog signal $e_1$, $e_2$ from a respective electrode 30 and to perform basic signal preconditioning of the respective analog signal. To this end, the analog section may include an analog filter 25 having a transfer function $H_i(s)$ where i denotes a channel number. The analog filter 25 may be designed as an anti-aliasing filter to prevent aliasing effects when digitizing the analog signal and/or may be designed to adapt a voltage or energy range of the respective signal $e_i$ to an input range of an analog-to-digital converter (ADC 27) connected to the analog filter 25. The ADC 27 is operable for converting an analog signal $e_{hi}$ outputted by the analog filter 25 of the analog section into a corresponding digital signal $d_i$, thereby forming an interface between the analog section of the respective channel 23 and a digital section of this channel 23. The digital section of the channel 23 includes a digital filter 29 adapted to filter the digital signal $d_i$ to obtain a filtered digital signal $d_{fi}$. The digital filter 29 may include a Finite Impulse Response (FIR) filter defined by a set of filter coefficients $G_i$.

In the example shown in Fig. 1, one ADC 27 is provided for each channel 23. However, it is also possible to implement less ADCs 27 in the front end 15 than channels 23. In other words, at least one ADC may be shared among multiple channels 23. In a practical implementation, one ADC may include an analog input multiplexer, inputs of which being connected to outputs of the analog filters 25 of different channels 23 to sequentially forward the individual filtered analog signals $e_{hi}$ to the ADC so that these signals $e_{hi}$ are sequentially sampled and digitized by the shared ADC. Sequential sampling and digitizing typically introduces a time skew between the digital signals $d_i$ of different channels 23 since the respective analog signals $e_i$ are sampled successively at different time instances.

The front end 15 further includes a combiner 31 configured to combine at least two filtered digital signals $df_i$, thereby obtaining a vector signal $x_1$. In the shown example, the combiner 31 is adapted to subtract the second filtered digital signal $df_2$ from the first digital signal $df_1$. Accordingly, weights used to calculate the vector signal $x_1$ are 1, and -1, respectively.

The signal processing front end 15 further comprises a front end processor 33. The processor 33 may include a programmable digital computer 35 including a memory device 37. A computer program may be stored on the memory device 37 when is programmed to cause the computer 35 to execute the herein described processing operations. Such computer program may also be stored on any storage device (flash storage, magnetic storage, etc.) that is not part of the processor 33. A program may be even made available for download over a communication network such as the internet. It should also be noted that in some implementations signal processing operations performed by blocks of Fig. 1 shown outside of the processor 33, in particular operations performed by the digital section of the input channels 23, may also be performed by the computer 35 of the processor 33.

The front end processor 33 is configured, e.g. programmed, to receive the vector signal $x_1$ and to calculate the sets $G_1$, $G_2$ of filter coefficients for the digital filters 29 based on the vector signal. Furthermore, the processor 33 may calculate the sets of filter coefficients also based on samples and $d_1$, $d_2$ of the unfiltered digital signal output by the ADC 27 of individual channels 23. Moreover, the processor 33 may be configured to calculate the sets of coefficients of filters 29 iteratively; that is the calculation of the sets of filter coefficients will further be based on current sets of filter coefficients.

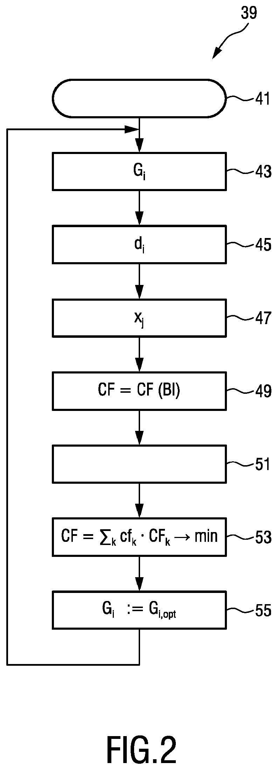

Fig. 2 shows a method 39 that can be carried out by the front end processor 33. In particular, the program stored in the memory device 37 may be programmed to execute the method 39.

After a start 41 of the method, a step 43 is executed for reading current sets of filter coefficients $G_1$, ..., $G_N$, where N denotes the number of channels 23. In the example shown in Fig. 1, the number of channels is two thus N = 2.

Step 45, which may follow step 43, reads a sequence of samples of the unfiltered digital signal where $d_i$ is a sequence of samples of digital signal i, i = 1, ..., N. The unfiltered digital signal $d_i$ is the digital signal present in the respective channel 23 that has not yet been processed by the digital filter 29. For example, the unfiltered digital signal may correspond to the signal generated by the respective ADC 27. Step 45 may capture exactly one sample per channel 23 (on-line mode). Alternatively, step 45 may collect a sequence of multiple samples for each channel 23 (batch mode). For the sake of simplicity, the single sample of a certain channel 23 or the sequence of multiple samples of a certain channel e will be referred to as $d_i$. Accordingly, in step 45 the values $d_1$, ..., $d_N$ are obtained.

In a step 47 of the method 39, subsequent samples $x_j$ of one or more output vectors are $x_j$ collected. The example of Fig. 1 is only one vector signal $x_1$. However, in the general case, the method 39 may consider M vector signals $x_1$, ..., $x_M$. The number of samples collected for the individual vector signals $x_j$ is preferably so selected that statistical measures can be calculated from the individual sequences $x_j$ in a reasonable way.

Step 49 of the method 39 selects a cost function CF based on an indicator BI received from the back end processor 17. The indicator BI may include information determined by the back end processor 17 derived from long-term, high level signal analysis such as rhythm classification or results of automatic analysis or classification performed by the back end processor 17. The indicator BI thus includes information usable by the processor 33 to appropriately select and/or compose the cost function CF.

As will be described in detail below, the method 39 uses statistical properties of at least one vector signal $x_j$ to determine if and to what the extent the vector signal is adversely affected by disturbances such as common mode interference. Because the measurement device is used to process a wide range of normal and pathological waveforms, such as ECG waveforms, adapting the cost function CF to certain pathological patterns like tachycardia or ventricular fibrillation can improve the performance of the method 39.

In an ECG recorder, for instance, the component responsible for signal acquisition and conditioning (front end 15) may be not aware of this long-term signal analysis. Thanks to the indicator BI received from the front end processor, the cost function CF can be adapted to best distinguish signals corresponding to the currently identified cardiac rhythm from common-mode interference sources. As an alternative to the dynamic cost function selection, adaptation or composition of step 49, a fixed cost function CF may be used and step 49 may be omitted.

The cost function CF is used to measure the statistical similarity of a vector signal $x_j$ with a desired signal such as an ECG signal, or its dissimilarity with expected common mode interference signals of other disturbances. Components of the cost function may include statistical measures of the (numerically calculated) derivative of the vector signal. The derivative considers the time structure, whereas measures like kurtosis and skewness consider the histogram and are unchanged when the order of the samples is changed. As an example, a measure that quantifies the similarity of the distribution of the vector signal to that of a sine wave would show a high amount of similarity when the input signal is a random permutation of the samples of a sine wave, but the difference would become clear if the derivative of the vector signal is considered.

In an implementation, the values of the vector signal used for calculating the statistical measures may be normalized first, i.e. shifted so their mean is zero, and scaled so their standard deviation is one. Depending on the number of available samples, the normalization may be approximate. Normalization makes the calculated measures (and their derivatives) scale-invariant.

In the following, possible statistical measures to be calculated based on the collected subsequent samples of the vector signal determined in step 47 will be described.

To measure the density of a vector signal $x_j$ the L 1-norm of $x_j$ may be used. For example, a vector signal $x_j$ used in an ECG application has usually a sparse density where many samples are at least close to zero except for some pathological patterns. A common mode signal, however is dense because it usually has a sinusoidal periodic or quasi-periodic pattern.

To measure the total variation of the vector signal $x_j$ over time the L1-norm of the differentiated vector signal $x_j$ may be used. The differentiated vector signal can be calculated by subtracting subsequent values from each other (by subtracting a respective preceding value from each value). The total variation of e.g. an ECG vector signal is low, whereas a total variation of a common mode interfering signal is high.

To measure an amplitude, the L2-norm (Euclidian norm) or the L∞-norm may be used. Desired ECG signals have a limited amplitude that is typical in the range below 10 mV p-p whereas the amplitude of a disturbing common mode signal is often larger than 10 mV p-p.

Moreover, measures related to a probability density distribution of the vector signal $x_j$ can be used. When using the Kurtosis of the probability density distribution as a cost function, a vector signal usually having significant supergaussian/leptokurtic properties can be distinguished from disturbing common mode signals usually having subgaussian/platykurtic properties.

As an alternative to the Kurtosis or in addition to the Kurtosis, the negentropy measure (or an approximation thereof) calculated for the probability density distribution can be used as a cost function.

To measure the symmetry of the probability density distribution, the skewness measure of the probability density function may be used as a cost function. Instead of the skewness, an asymmetry heuristics that is more robust to outliers may be used such as $x_j*\exp(-x_j^2/2)$.

One of the above-mentioned cost functions can be used as the cost functions CF do calculate optimized sets $G_i$ of filter coefficients. Alternatively, the cost function CF may be composed by a weighted addition of multiple cost functions, $CF = \sum_{k=1}^{K} cf_k \cdot CF_k$, where $CF_k$ is one cost function, $cf_k$ the respective weight and K the number of cost functions $CF_k$ used to compose the final cost function CF. For example, the overall cost function CF could be chosen to be the L1-norm minus skewness of a resulting vector $x_j$. A negative gradient (considering the optimized sets $G_i$ of filter coefficients to be determined as variables) would then be a direction that minimizes the L1-norm (maximizing sparsity) but also maximizes skewness. For example, the weights $cf_k$ may be determined based on the indicator BI so that the cost function CF is selected/composed from/of a set of K predefined cost functions $CF_k$. Selecting a certain cost function $CF_k$ can be carried out by setting the respective weight to any value $cf_k \neq 0$. To deselect a certain cost function $CF_k$, the processor 33 may set the respective weight to the value $cf_k = 0$.

The method 39 may comprise a step 51 for defining one or more constraints to be used when optimizing the sets $G_i$ of filter coefficients. Such constraints, e.g. in the form of equalities or inequalities, place limits on the solution space, i.e. the mathematical domain in which the optimization algorithm used in a later step of the method 39 can search for an optimum value of the cost function CF.

For example, an equality constraint requiring the sum of the coefficients $G_i$ to be constant may be used to force the DC gain (gain at 0 Hz) of a filter 29 to be constant. A constraint of this type can be used when it is known that the gain at low frequency will not change when the measurement device 11 is in operation and forces the optimization algorithm to change the frequency response at higher frequencies only. Similarly, another constraint could be applied that forces the gain of a filter 29 at frequency other than DC to a constant value.

Furthermore, constraints can be defined to prevent one or more digital filters 29 of different channels 23 from becoming too similar to each other and attenuating the same frequency band of input signals $e_i$ while attempting to find the optimum point of the cost function CF. This could happen in a situation where the common mode signal is dominated by a single frequency (e.g. powerline frequencies of 50 Hz or 60 Hz). The optimization algorithm could place a zero in the frequency response of the individual digital filters 29 and thus remove the common-mode interference even before the vector signal $x_j$ is calculated, e.g. by subtracting two digital signals $d_i$ from each other. Removing the common-mode part by band-stop filtering rather than subtracting is not the goal of electrode equalization and would have a significant adverse effect on the difference signal of interest. This constraint may include a similarity measure to be calculated for the sets of filter coefficients of the respective digital filters. Instead of, or in addition to the similarity measure, gain constraints in inequality form may be used for frequencies inside the range of the signal of interest to avoid band-stop behavior.

Additionally or alternatively to using constraints to prevent the method 39 from choosing similar filter coefficients for different filters 29, the similarity measure related to the similarity of the filters 29 may also be included into the cost function CF.

Constraints may also be defined in order to limit some norm of the pulses of one or more filter 29 to a given value. Limiting this norm has the effect that the numerical stability of the filters 29 is improved by avoiding numerical overflow or saturation.

Based on the selected or composed target function CF and the constraints defined in step 51, step 53 of the method 39 performs an optimization operation to calculate new sets $G_{i,opt}$ of filter coefficients from the vector signal $x_j$ the sample (on-line mode) or sequence of samples (batch mode) of the unfiltered digital signal $d_i$ and the vector signal $x_j$. The new filter coefficients $G_{i,opt}$ constitute variables of an optimization problem to be solved in step 53. Because the delay caused by the digital filters 29 can be quite easily controlled (compared to analog filters) by appropriately choosing the set of coefficients $G_i$, the optimization step 51 will find a solution that reduces any adverse effect of the above-described time skew between digital signals $d_i$, on the effectiveness of common-mode interference mitigation.

When operating in on-line mode, known minimization algorithms such as stochastic gradient descent, in particular least mean squares (LMS) or recursive least squares (RLS) may be applied. In batch mode, known minimization algorithms such as deepest descent, Newton iteration or inner-point methods may be applied. Step 53, however, is not limited to a certain type of optimization algorithm. Although the optimization problem is formulated here as a cost minimization problem, an equivalent utility maximization problem may be formulated in steps 49 and 51 and solved in step 53.

After completion of the optimization run in step 53, the obtained new filter coefficients $G_{i,opt}$ may be applied to the individual digital filters 29 in step 55.

After step 55, the method 39 goes back to step 43 so that steps 43 to 55 are carried out iteratively either for each sample (on-line mode) or for the sequence of subsequent samples (batch mode) of the digital signals $d_i$.

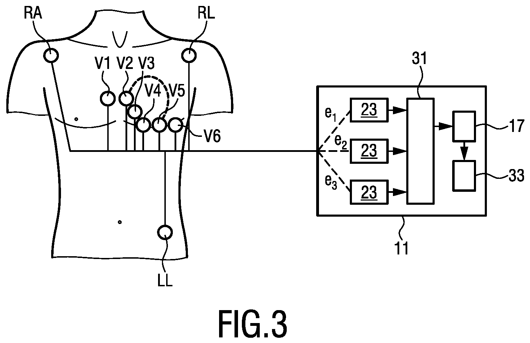

Although the herein described principle of the measurement device 11 and method 39 can be applied to any type of electrophysiological measurement procedures like EEG or EMG, in the following a more detailed example related to ECG measurements is given. This example includes a free channel ECG measurement device 11, the channels corresponding to three limb electrodes (right arm RA, left arm LA, left leg LL). As illustrated in Fig. 3, the three electrodes LA, LA, LL correspond to the analog electrical signals e1, e2, e3, respectively. This example can be extended as will be described below by adding further channels and electrodes such as six chest electrodes (V1, ..., V6).

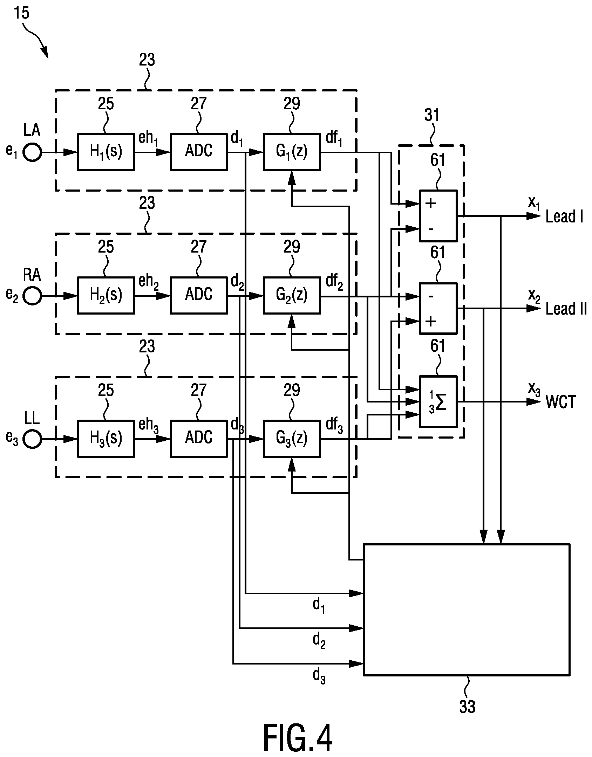

Fig. 4 shows a block diagram of the signal acquisition front end 15 of the three channel ECG device illustrated in Fig. 3. The three channel front end 15 has the same structure as the front end 15 shown in Fig. 1 but a further channel 23 has been added. Due to the higher number of channels, more than one vector signal $x_i$ may be defined. In the here described ECG application, three vector signals $x_1$, $x_2$, $x_3$ correspond to three leads referred to as "Lead I", "Lead II" and "WCT", respectively. WCT stands for the Wilson Central Terminal, which is a virtual potential calculated as the average of LA, RA and LL. In the shown example, the processor 33 performs optimization for the two leagues $x_1$, $x_2$ simultaneously, thereby optimizing the coefficients $G_i$ of filters 29 of all channels 23 that are used to calculate the respective vector signals $x_1$, $x_2$. Although at least one channel 23, such as the channel 23 related to the input signal $e_2$ and the electrode RA, is used to obtain multiple vector signals, there is only one channel filter 29 assigned to that channel. In other words, different vector signals $x_j$ that are determined based on the digital signal $df_j$ of the same channel 23 share the digital filter 23 of that channel.

As can be seen in Fig. 4, the combiner 35 as three separate combining elements 61 assigned to the individual vector signals $x_j$. In particular, $x_1$ (Lead I) is calculated by subtracting the digital signal $df_2$ related to the electrode RA of the digital signal $df_1$ related to the electrode LA. The vector signal $x_2$ is calculated by subtracting the digital signal $df_2$ related to the electrode RA from the digital signal $df_3$ related to the electrode LL.

Furthermore, it should be noted that it is not necessary to apply the above-described optimization method 39 to each vector signal $x_j$. In the shown example, the vector signal $x_3$ (WCT) is excluded from optimization, in particular the cost function CF is not calculated based on this vector signal X3.

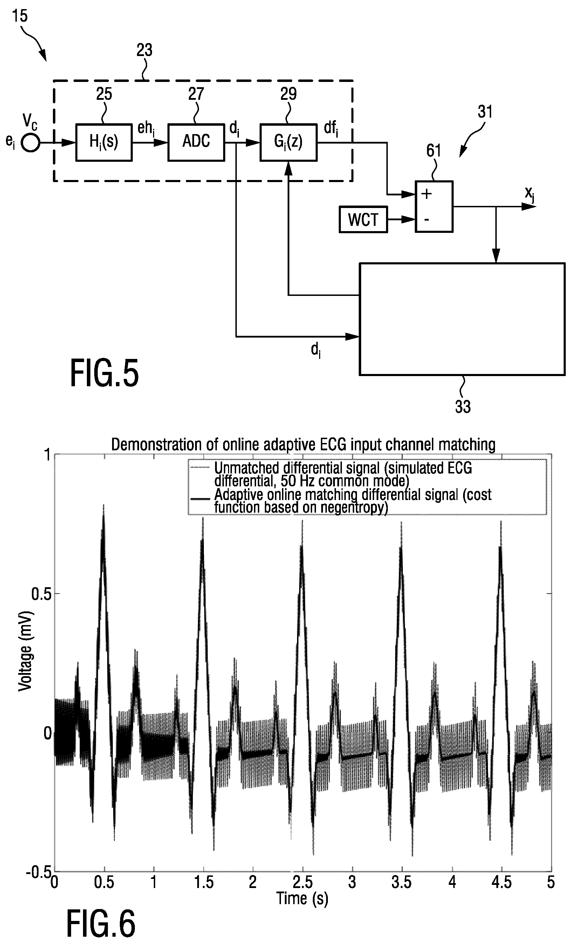

In a further example depicted on Fig. 5, the measurement device 11 shown in Fig. 3 and Fig. 4 is extended so that it can calculate further vector signals $x_j$, $j = 4, ..., M$ based on electrical signals $e_i$, $i = 4, ..., M$ inputted from the chest electrodes V1-V6. Accordingly, the front end 15 comprises at least one additional channel 23 assigned to one chest electrode. In case of a nine channel ECG device, six additional channels 23 are provided for the individual chest electrodes V1 to V6. Respective combining elements 61 of the signal combiner 31 calculate the difference between the filtered digital signal $df_i$ of the respective channel 23 and the virtual potential WCT corresponding to the vector signal $x_3$. For the sake of simplicity only one of these additional channels 23 and one respective combining element 61 is shown in Fig. 5. The respective vector signal $x_j$ is a further variable of the cost function CF and the respective unfiltered digital signal $d_i$ is used for calculating an optimized version of the respective filter coefficients $G_j$. No filtering is applied to the WCT-related vector signal $x_3$. The processor 33 performs the optimization method 39 described above and shown in Fig. 2. However, compared to the example of Fig. 1, the complexity of the optimization problem is increased due to the higher number of sets of filter coefficients $G_i$ and the increased number of vector signals $x_j$.

As can be seen in Fig. 4 and 5, the signal combiner 31 may include multiple combining elements 61. To calculate at least one vector signal $x_j$ from at least two processed digital signals $df_i$, a single combining element 61 may be used (e.g. for calculating the vector signals x1, x2, x3 corresponding to Lead I, Lead II and WCT) or multiple combining elements 61 may be combined, in particular cascaded (e.g. the vector signals xj shown in Fig. 5 related to the chest leads are calculated by the combining element 61 of Fig. 6 and the WCT combining element 61 shown in the bottom of Fig. 4). Combining and/or cascading combining elements 61 is particularly useful when different vector signals need to be calculated by the signal combiner 31 from a common vector signal such as the vector signal $x_3$ corresponding to the WCT.

Fig. 6 shows how the method 39 improves the signal quality when performing on-line optimization in the course of a measurement process. More specifically, the diagram of Fig. 6 shows the voltage in millivolt of a vector signal over time (in seconds). The vector signal obtained without applying the adaptive filters 29 is shown as a grey curve. The vector filter obtained when applying the filters 29 in the individual channels 23 is shown as a black curve in the diagram. As can be seen, the latter signal is superimposed by disturbing noise due to common mode interference at the beginning of the measurement (time t = 0). In the curse of the measurement the proportion of the disturbing noise gradually decreases while the method 39 continuously adapts the sets of filter coefficients $G_i$ of the digital filters 29.

To sum up, the herein described method 39 and the respective devices 11, 15 allow to iteratively adapt digital filters 29 present in the individual input channels 23 in order to reduce disturbances such as common mode interference. The filter coefficients $G_i$ are adapted based on a cost function related on statistical properties of at least one vector signal $x_j$. The electrode-specific adaptive filters 29 are iteratively tuned based on an optimization algorithm using a cost function composed of statistical properties of at least one vector signal, for example L1-norm, total variation, kurtosis, skewness or negentropy. The inventor has realized that good common-mode rejection can be obtained by minimizing the statistical similarity of the vector signal with usual common-mode signal patterns and/or maximizing the statistical similarity of the at least one vector signal with expected patterns of a desired signal such as expected ECG signal patterns.

The method disclosed in EP 3372148 A1 relies on properties of the device that are measured while the device is not actively performing measurements (i.e. using test signals), and they can only compensate effects that are measurable at this time. Effects that only occur when the device is connected to a patient and performing measurements, such as the electrical behavior of the skin-electrode interface, cannot be compensated this way.

As explained above, in physiological measurements the signal path of each electrode has a slightly different transfer function due to factors like component tolerances, different external cables, and the electrical properties of the skin-electrode interface. If the different transfer functions are not compensates for, this results in the common-mode signal only being partially cancelled out by forming the difference between two electrode potentials. The part of the common-mode signal not being cancelled out appears in the difference signal and impedes the analysis of the physiological signal for diagnostic purposes. Part of the transfer function asymmetry is intrinsic to each device and can be compensated by a calibration process (e.g. as disclosed in EP 3372148 A1) before the devices are sold to customers. However, any part of the asymmetry that is external to the device - like different cables or the properties of the skin-electrode interface - is not known at the time of production and cannot be compensated by factory calibration. Furthermore, the asymmetry can slowly change over time as the adhesive electrodes attaches to the patient dry out or the local conductivity of the patient's skin changes. If too much of the common-mode signal leaks into the difference-mode vectors, the diagnostic value of the resulting wave is diminished. Medical conditions may remain undiagnosed or be misdiagnosed, or analysis of the signal (including automated analysis) may become impossible.

According to the present invention, in contrast to the known solutions, in particular the solution disclosed in EP 3372148 A1, no test (or reference) signals are used, but the input signals are measured/sensed when the electrodes are actually attached to the patient and the device is actually in use. In this way, the disclosed device and method can automatically compensate for the electrode transfer function asymmetries, even without the use of test (or reference) signals injected into the patient or the electrodes. In embodiments, differences in statistical properties of the physiological signal and usual common-mode interference signals may be used to compute or adapt the coefficients of the digital filters. The computation and adaptation of the filter coefficients of the electrode-specific filter aims to minimize or maximize a cost function that reflects certain statistical properties of the vectors which allow distinguishing between physiological signals and non-physiological interference. This compensates the input transfer function differences between the electrodes and increases the degree to which non-physiological common-mode signal components are cancelled out.

**Claims**

1. Physiological measurement device (11) comprising:

   a plurality of input channels (23), at least two of the input channels each including a digital section configured to process a digital signal $(d_1, d_2)$ representing an analog signal $(e_1, e_2)$ present at an electrode (13) assigned to the respective channel and arranged to pick up a physiological signal of interest, wherein the digital section of each of the at least two input channels (23) comprises an adaptive digital filter (29) configured to filter the digital signal $(d_1, d_2)$ based on a set of filter coefficients $(G_1, G_2)$,
   a signal combiner (31) configured to calculate at least one vector signal (x) from at least two processed digital signals $(df_1, df_2)$, wherein the vector signal is a linear combination where the sum of weighting coefficients is zero, and
   a processor (33) configured to calculate the set of filter coefficients $(G_1, G_2)$ of the adaptive digital filters (29) of the digital section of each of the at least two input channels (23) based on the vector signal $(x_1)$.

2. Device (11) according to claim 1, wherein calculating the set of filter coefficients comprises calculating (49) at least one measure, preferably a statistical measure, of a block of subsequent time domain samples of the vector signal (x).

3. Device (11) according to claim 1 or 2, wherein calculating the set of filter coefficients $(G_1, G_2)$ is further based on the unfiltered digital signal $(d_1, d_2)$.

4. Device (11) according to any one of the preceding claims, wherein calculating the set of filter coefficients is further based on a current set of filter coefficients (G1, G2) and wherein calculating the set of filter coefficients $(G_1, G_2)$ is performed repeatedly for each newly available sample of the digital signal $(d_1, d_2)$ or for a block of multiple subsequent samples of the digital signal $(d_1, d_2)$.

5. Device (11) according to any one of the preceding claims, wherein calculating the set of filter coefficients $(G_1, G_2)$ comprises performing (53) an optimization step, the measure constituting a target function (CF) and the set of filter coefficients $(G_1, G_2)$ constituting input variables of the target function.

6. Device (11) according to claim 5, wherein the target function (CF) comprises a weighted sum of multiple measures $(CF_K)$.

7. Device (11) according to claim 5 or 6, wherein performing (53) the optimization step is subject to at least one constraint related to a certain set of filter coefficients $(G_1, G_2)$, in particular a constraint that includes at least one equality constraint and/or at least one inequality constraint.

8. Device (11) according to any one of claims 5 to 7, wherein performing (53) the optimization step is subject to at least one constraint related to a similarity measure characterizing a similarity of two different sets of filter coefficients $(G_1, G_2)$.

9. Device (11) according to any one of claims 5 to 8, wherein the target function (CF) comprises the similarity measure characterizing a similarity of two different sets of filter coefficients.

10. Device (11) according to any one of the preceding claims, wherein the digital section is configured to process a digital signal $(d_1, d_2)$ representing an analog signal $(e_1, e_2)$ present at an electrode (13) assigned to the respective channel and sensed from a patient during use of the device.

11. Device (11) according to any one of the preceding claims, wherein the processor (33) is configured to calculate multiple vector signals $(x_1, x_2, x_3, x_j)$ from the filtered digital signals $(df_1, df_2, df_3, df_i)$, wherein the digital section of one channel (23) includes at most one adaptive digital filter (29).

12. Device (11) according to any one of the preceding claims, wherein the processor (32) is configured to receive (49) an indication (BI) from a back end (17) and to determine the at least one measure and/or the target function based on the received indication.

13. Device (11) according to any one of the preceding claims, wherein the signal combiner (31) is configured to calculate the vector signal $(x_j)$ from a further vector signal $(x_3)$.

14. Physiological measurement method (39) comprising:

processing at least two digital signals $(d_1, d_2)$ each representing an analog signal $(e_1, e_2)$ present at a respective electrode (13) assigned to a respective digital section of a respective one of at least two input channels (23) of a physiological measurement device (11) and arranged to pick up a physiological signal of interest, wherein processing the digital signal $(d_1, d_1)$ comprises adaptively digitally filtering the digital signal $(d_1, d_2)$ of the respective input channel (23) based on a set of filter coefficients $(G_1, G_2)$ of a respective adaptive digital filter (29);
calculating at least one vector signal (x) from at least two processed digital signals $(df_1, df_2)$, wherein die vector signal is a linear combination where the sum of weighting coefficients is zero; and
calculating the set of filter coefficients $(G_1, G_2)$ of the adaptive digital filters (29) of the digital section of each of the at least two input channels (23) based on the vector signal (x).

15. Computer program comprising program code means (37) for causing a computer (35) to carry out the steps of the method (39) as claimed in claim 14 when said computer program is carried out on the computer (35).

**Patentansprüche**

1. Physiologische Messvorrichtung (11), umfassend:

eine Vielzahl von Eingangskanälen (23), wobei mindestens zwei der Eingangskanäle jeweils einen digitalen Abschnitt beinhalten, der dazu konfiguriert ist, ein digitales Signal $(d_1, d_2)$ zu verarbeiten, das ein analoges Signal $(e_1, e_2)$ darstellt, das an einer dem jeweiligen Kanal zugewiesenen Elektrode (13) anliegt und dazu eingerichtet ist, ein physiologisches Signal von Interesse aufzunehmen, wobei der digitale Abschnitt jedes der mindestens zwei Eingangskanäle (23) ein adaptives digitales Filter (29) umfasst, das dazu konfiguriert ist, das digitale Signal $(d_1, d_2)$ basierend auf einem Satz von Filterkoeffizienten $(G_1, G_2)$ zu filtern,
einen Signalkombinierer (31), der dazu konfiguriert ist, aus mindestens zwei verarbeiteten digitalen Signalen $(df_1, df_2)$ mindestens ein Vektorsignal (x) zu berechnen, wobei das Vektorsignal eine lineare Kombination ist, bei der die Summe von Gewichtungskoeffizienten Null ist, und
einen Prozessor (33), der dazu konfiguriert ist, den Satz von Filterkoeffizienten $(G_1, G_2)$ der adaptiven digitalen Filter (29) des digitalen Abschnitts jedes der mindestens zwei Eingangskanäle (23) basierend auf dem Vektorsignal $(x_1)$ zu berechnen.

2. Vorrichtung (11) nach Anspruch 1, wobei Berechnen des Satzes von Filterkoeffizienten Berechnen (49) mindestens eines Maßes, vorzugsweise eines statistischen Maßes, eines Blocks aufeinanderfolgender Zeitbereichsabtastwerte des Vektorsignals (x) umfasst.

3. Vorrichtung (11) nach Anspruch 1 oder 2, wobei Berechnen des Satzes von Filterkoeffizienten $(G_1, G_2)$ weiter auf dem ungefilterten digitalen Signal $(d_1, d_2)$ basiert.

4. Vorrichtung (11) nach einem der vorstehenden Ansprüche, wobei Berechnen des Satzes von Filterkoeffizienten weiter auf einem aktuellen Satz von Filterkoeffizienten (G1, G2) basiert und wobei Berechnen des Satzes von Filterkoeffizienten $(G1,G2)$ wiederholt für jeden neu verfügbaren Abtastwert des digitalen Signals $(d1,d2)$ oder für einen Block mehrerer aufeinanderfolgender Abtastwerte des digitalen Signals $(d1,d2)$ durchgeführt wird.

5. Vorrichtung (11) nach einem der vorstehenden Ansprüche, wobei Berechnen des Satzes von Filterkoeffizienten $(G_1, G_2)$ Durchführen (53) eines Optimierungsschritts umfasst, wobei das Maß eine Zielfunktion (CF) bildet und der Satz von Filterkoeffizienten $(G_1, G_2)$ Eingangsvariablen der Zielfunktion bildet.

6. Vorrichtung (11) nach Anspruch 5, wobei die Zielfunktion (CF) eine gewichtete Summe mehrerer Maße $(CF_K)$ umfasst.

7. Vorrichtung (11) nach Anspruch 5 oder 6, wobei Durchführen (53) des Optimierungsschritts mindestens einer Beschränkung in Bezug auf einen bestimmten Satz von Filterkoeffizienten $(G_1, G_2)$ unterliegt, insbesondere einer Beschränkung, die mindestens eine Gleichheitsbeschränkung und/oder mindestens eine Ungleichheitsbeschränkung beinhaltet.

8. Vorrichtung (11) nach einem der Ansprüche 5 bis 7, wobei Durchführen (53) des Optimierungsschritts mindestens einer Beschränkung unterliegt, die sich auf ein Ähnlichkeitsmaß bezieht, das eine Ähnlichkeit zweier unterschiedlicher Sätze von Filterkoeffizienten $(G_1, G_2)$ charakterisiert.

9. Vorrichtung (11) nach einem der Ansprüche 5 bis 8, wobei die Zielfunktion (CF) das Ähnlichkeitsmaß umfasst, das eine Ähnlichkeit zweier unterschiedlicher Sätze von Filterkoeffizienten charakterisiert.

10. Vorrichtung (11) nach einem der vorstehenden Ansprüche, wobei der digitale Abschnitt dazu konfiguriert ist, ein digitales Signal ($d_1$, $d_2$) zu verarbeiten, das ein analoges Signal ($e_1$, $e_2$) darstellt, das an einer dem jeweiligen Kanal zugeordneten Elektrode (13) anliegt und während Verwendung der Vorrichtung von einem Patienten erfasst wird.

11. Vorrichtung (11) nach einem der vorstehenden Ansprüche, wobei der Prozessor (33) dazu konfiguriert ist, aus den gefilterten digitalen Signalen ($df_1$, $df_2$, df3, $df_i$) mehrere Vektorsignale ($x_1$, $x_2$, $x_3$, $x_j$) zu berechnen, wobei der digitale Abschnitt eines Kanals (23) höchstens ein adaptives digitales Filter (29) beinhaltet.

12. Vorrichtung (11) nach einem der vorstehenden Ansprüche, wobei der Prozessor (32) dazu konfiguriert ist, eine Anzeige (BI) von einem Back-End (17) zu empfangen (49) und das mindestens eine Maß und/oder die Zielfunktion basierend auf der empfangenen Anzeige zu bestimmen.

13. Vorrichtung (11) nach einem der vorstehenden Ansprüche, wobei der Signalkombinierer (31) dazu konfiguriert ist, das Vektorsignal ($x_j$) aus einem weiteren Vektorsignal ($x_3$) zu berechnen.

14. Physiologisches Messverfahren (39), umfassend:

Verarbeiten von mindestens zwei digitalen Signalen ($d_1$, $d_2$), die jeweils ein analoges Signal ($e_1$, $e_2$) darstellen, das an einer jeweiligen Elektrode (13) anliegt, die einem jeweiligen digitalen Abschnitt eines jeweiligen von mindestens zwei Eingangskanälen (23) einer physiologischen Messvorrichtung (11) zugeordnet ist und zum Aufnehmen eines interessierenden physiologischen Signals eingerichtet ist, wobei Verarbeiten des digitalen Signals ($d_1$, $d_2$) adaptives digitales Filtern des digitalen Signals ($d_1$, $d_2$) des jeweiligen Eingangskanals (23) basierend auf einem Satz von Filterkoeffizienten ($G_1$, $G_2$) eines jeweiligen adaptiven digitalen Filters (29) umfasst;
Berechnen mindestens eines Vektorsignals (x) aus mindestens zwei verarbeiteten digitalen Signalen ($df_1$, $df_2$), wobei das Vektorsignal eine lineare Kombination ist, bei der die Summe von Gewichtungskoeffizienten Null ist; und
Berechnen des Satzes von Filterkoeffizienten ($G_1$, $G_2$) der adaptiven digitalen Filter (29) des digitalen Abschnitts jedes der mindestens zwei Eingangskanäle (23) basierend auf dem Vektorsignal (x).

15. Computerprogramm, das Programmcodemittel (37) umfasst, um einen Computer (35) zu veranlassen, die Schritte des Verfahrens (39) nach Anspruch 14 auszuführen, wenn das Computerprogramm auf dem Computer (35) ausgeführt wird.

## Revendications

1. Dispositif de mesure physiologique (11) comprenant :

une pluralité de canaux d'entrée (23), au moins deux des canaux d'entrée incluant chacun une section numérique configurée pour traiter un signal numérique ($d_1$, $d_2$) représentant un signal analogique ($e_1$, $e_2$) présent sur une électrode (13) assignée au canal respectif et agencée pour capter un signal physiologique d'intérêt, dans lequel la section numérique de chacun des au moins deux canaux d'entrée (23) comprend un filtre numérique adaptatif (29) configuré pour filtrer le signal numérique ($d_1$, $d_2$) sur la base d'un ensemble de coefficients de filtre ($G_1$, $G_2$),
un combineur de signaux (31) configuré pour calculer au moins un signal de vecteur (x) à partir d'au moins deux signaux numériques traités ($df_1$, $df_2$), dans lequel le signal de vecteur est une combinaison linéaire où la somme de coefficients de pondération est nulle, et
un processeur (33) configuré pour calculer l'ensemble des coefficients de filtre ($G_1$, $G_2$) des filtres numériques adaptatifs (29) de la section numérique de chacun des au moins deux canaux d'entrée (23) sur la base du signal de vecteur ($x_1$).

2. Dispositif (11) selon la revendication 1, dans lequel le calcul de l'ensemble de coefficients de filtre comprend le calcul (49) d'au moins une mesure, de préférence une mesure statistique, d'un bloc d'échantillons de domaine temporel ultérieurs du signal de vecteur (x).

3. Dispositif (11) selon la revendication 1 ou 2, dans lequel le calcul de l'ensemble de coefficients de filtre ($G_1$, $G_2$) est en outre basé sur le signal numérique non filtré ($d_1$, $d_2$).

4. Dispositif (11) selon l'une quelconque des revendications précédentes, dans lequel le calcul de l'ensemble de coefficients de filtre est en outre basé sur un ensemble actuel de coefficients de filtre (G1, G2) et dans lequel le calcul de l'ensemble de coefficients

de filtre ($G_1$, $G_2$) est réalisé de manière répétée pour chaque échantillon nouvellement disponible du signal numérique ($d_1$, $d_2$) ou pour un bloc de multiples échantillons ultérieurs du signal numérique ($d_1$, $d_2$).

5. Dispositif (11) selon l'une quelconque des revendications précédentes, dans lequel le calcul de l'ensemble de coefficients de filtre ($G_1$, $G_2$) comprend la réalisation (53) d'une étape d'optimisation, la mesure constituant une fonction cible (CF) et l'ensemble de coefficients de filtre ($G_1$, $G_2$) constituant des variables d'entrée de la fonction cible.

6. Dispositif (11) selon la revendication 5, dans lequel la fonction cible (CF) comprend une somme pondérée de multiples mesures ($CF_K$).

7. Dispositif (11) selon la revendication 5 ou 6, dans lequel la réalisation (53) de l'étape d'optimisation est soumise à au moins une contrainte liée à un certain ensemble de coefficients de filtre ($G_1$, $G_2$), en particulier une contrainte qui inclut au moins une contrainte d'égalité et/ou au moins une contrainte d'inégalité.

8. Dispositif (11) selon l'une quelconque des revendications 5 à 7, dans lequel la réalisation (53) de l'étape d'optimisation est soumise à au moins une contrainte liée à une mesure de similarité caractérisant une similarité de deux ensembles différents de coefficients de filtre ($G_1$, $G_2$).

9. Dispositif (11) selon l'une quelconque des revendications 5 à 8, dans lequel la fonction cible (CF) comprend la mesure de similarité caractérisant une similarité de deux ensembles différents de coefficients de filtre.

10. Dispositif (11) selon l'une quelconque des revendications précédentes, dans lequel la section numérique est configurée pour traiter un signal numérique ($d_1$, $d_2$) représentant un signal analogique ($e_1$, $e_2$) présent au niveau d'une électrode (13) assignée au canal respectif et détecté à partir d'un patient pendant l'utilisation du dispositif.

11. Dispositif (11) selon l'une quelconque des revendications précédentes, dans lequel le processeur (33) est configuré pour calculer de multiples signaux de vecteur ($x_1$, $x_2$, $x_3$, $x_j$) à partir des signaux numériques filtrés ($df_1$, $df_2$, $df3$, $df_i$), dans lequel la section numérique d'un canal (23) inclut au plus un filtre numérique adaptatif (29).

12. Dispositif (11) selon l'une quelconque des revendications précédentes, dans lequel le processeur (32) est configuré pour recevoir (49) une indication (BI) d'un back-end (17) et pour déterminer l'au moins une mesure et/ou la fonction cible sur la base de l'indication reçue.

13. Dispositif (11) selon l'une quelconque des revendications précédentes, dans lequel le combineur de signaux (31) est configuré pour calculer le signal de vecteur ($x_j$) à partir d'un signal de vecteur supplémentaire ($x_3$).

14. Dispositif de mesure physiologique (39) comprenant :

le traitement d'au moins deux signaux numériques ($d_1$, $d_2$) représentant chacun un signal analogique ($e_1$, $e_2$) présent au niveau d'une électrode respective (13) assignée à une section numérique respective d'un canal respectif parmi au moins deux canaux d'entrée (23) d'un dispositif de mesure physiologique (11) et agencé pour capter un signal physiologique d'intérêt, dans lequel le traitement du signal numérique ($d_1$, $d_2$) comprend le filtrage numérique adaptatif du signal numérique ($d_1$, $d_2$) du canal d'entrée respectif (23) sur la base d'un ensemble de coefficients de filtre ($G_1$, $G_2$) d'un filtre numérique adaptatif respectif (29) ;
le calcul d'au moins un signal de vecteur (x) à partir d'au moins deux signaux numériques traités ($df_1$, $df_2$), dans lequel le signal de vecteur est une combinaison linéaire où la somme de coefficients de pondération est nulle, et
le calcul de l'ensemble des coefficients de filtre ($G_1$, $G_2$) des filtres numériques adaptatifs (29) de la section numérique de chacun des au moins deux canaux d'entrée (23) sur la base du signal de vecteur (x).

15. Programme informatique comprenant des moyens de code de programme (37) pour amener un ordinateur (35) à exécuter les étapes du procédé (39) selon la revendication 14 lorsque ledit programme informatique est exécuté sur l'ordinateur (35).

FIG.1

FIG.2

FIG.3

FIG.4

FIG.5

Demonstration of online adaptive ECG input channel matching

- Unmatched differential signal (simulated ECG differential, 50 Hz common mode)
- Adaptive online matching differential signal (cost function based on negentropy)

FIG.6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- DE 102014214994 A1
- EP 3372148 A1
- EP 2394571 A1
- US 4793361 A
- WO 2014021886 A1

### Non-patent literature cited in the description

- **ADAM et al.** Complete foetal ECG morphology recording by synchronised adaptive filtration. *Medical and Biological Engineering and Computing*, 1990, vol. 28 (4), 287-292